(19) Europäisches Patentamt
European Patent Office
Office européen des brevets

(11) **EP 1 433 476 B2**

(12) **NEW EUROPEAN PATENT SPECIFICATION**
After opposition procedure

(45) Date of publication and mention
of the opposition decision:
**24.07.2013 Bulletin 2013/30**

(45) Mention of the grant of the patent:
**22.03.2006 Bulletin 2006/12**

(51) Int Cl.:
*A61K 8/34* *(2006.01)*     *A61K 8/39* *(2006.01)*
*A61K 8/86* *(2006.01)*     *A61Q 19/10* *(2006.01)*
*A61Q 1/14* *(2006.01)*

(21) Application number: **03029675.0**

(22) Date of filing: **23.12.2003**

(54) **Skin cleansing composition**

Hautreinigungszusammensetzung

Composition de nettoyage pour la peau

(84) Designated Contracting States:
**DE FR GB**

(30) Priority: **27.12.2002 JP 2002379936**

(43) Date of publication of application:
**30.06.2004 Bulletin 2004/27**

(73) Proprietor: **KAO CORPORATION**
**Tokyo 103-8210 (JP)**

(72) Inventor: **Tomokuni, Atsushi,**
**Kao Corporation**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**EP-A- 0 620 270      EP-A- 1 053 740**
**EP-A1- 0 103 910     EP-A2- 0 217 105**
**WO-A2-02/26204       US-A- 5 474 776**
**US-A- 5 891 836**

- **DATABASE WPI Section Ch, Week 199502 Derwent Publications Ltd., London, GB; Class A96, AN 1995-009526 XP002296108 & JP 06 293617 A (LION CORP) 21 October 1994 (1994-10-21)**
- **DATABASE WPI Section Ch, Week 200245 Derwent Publications Ltd., London, GB; Class A96, AN 2002-420019 XP002296109 & JP 2002 020791 A (SHISEIDO CO LTD) 23 January 2002 (2002-01-23)**
- **DATABASE WPI Section Ch, Week 200065 Derwent Publications Ltd., London, GB; Class A26, AN 2000-667455 XP002296110 & JP 2000 256132 A (SHISEIDO CO LTD) 19 September 2000 (2000-09-19)**
- **DERMAL DELIVERY: ASSESSMENT OF BICONTINUOUS STRUCTURES": ' M.A. Thevenin et al.' INTERNATIONAL JOURNAL OF PHARMACEUTICS 137, 1996 "SUCROSE ESTERS/COSURFACTANT MICROEMULSION SYSTEMS FOR TRANS-, pages 177 - 186**
- **A. SPERNATH ET AL. J. AGRIC. FOOD CHEM., 2002 vol. 50, pages 6917 - 6922**

Remarks:
The file contains technical information submitted after the application was filed and not included in this specification

**EP 1 433 476 B2**

**Description**

**Technical Field**

The present invention relates to skin cleansing compositions.

**Background Art**

As cosmetic cleansing compositions, those containing an oil component have been employed for the removal of makeup cosmetics. They are provided as an emulsion type, a solubilization type or a liquid crystal type, depending on the combination with several surfactants. Of these, solubilization type compositions are particularly excellent from the viewpoints of spreadability and compatibility with stains.

Examples of the solubilization type compositions include those having an oil phase solubilized in an aqueous phase, those having water solubilized in an oil phase and those having both an oil phase and an aqueous phase in the continuous form. The first type having an oil phase solubilized in an aqueous phase has high cleansing ability for the removal of water soluble stains such as salts and water soluble polymers because the aqueous phase is continuous. However, its cleansing ability for the removal of oil soluble stains such as wax and liquid oil is not sufficient. The second type having water solubilized in an oil phase has high cleansing ability for the removal of oil soluble stains, but its cleansing ability for the removal of water soluble stains is not sufficient. The structure of the third type having both an oil phase and an aqueous phase in the continuous form is called "bicontinuous structure" and this type is expected to exhibit a high cleansing ability for the removal of both oil soluble stains and water soluble stains.

It is disclosed in "Journal of Oleo Science, Vol. 43, No. 2, p.131-136 (1994)" that when a composition comprising (1) hexane, (2) sodium oleate and (3) a monoalkylpolyoxyalkylene ether forms a bicontinuous structure, the time required for rolling up a model stain decreases. This composition is however not suited for use as a skin cleanser from the viewpoint of safety. In addition, the composition cannot be easily rinsed off by water.

In Japanese Patent Application Laid-Open No. Hei. 6-293617, it is disclosed that a nonaqueous cleansing composition comprising (1) a polar organic solvent, (2) an oil liquid incompatible with the polar organic solvent, and (3) a nonionic surfactant soluble in each of the polar organic solvent and the oil liquid, at a ratio of the components falling within a fishtail region (bicontinuous structure) has excellent temperature stability and can be easily removed by water after use. The composition however does not contain water so that it cannot provide users with cool and refreshing feeling, and therefore, is inferior in feeling upon use. It also involves a problem that the composition is not environmentally friendly because it is free of water.

In Japanese Patent Application Laid-Open No. 2002-20791, described is a composition which contains (1) an amphoteric surfactant, (2) an anionic surfactant, (3) a liquid alcohol and/or liquid fatty acid, and (4) water; satisfies the following relation: x<y-1 wherein x represents a surface tension of a 1 wt.% aqueous solution of (1) and (2) against decane, and y represents a smaller surface tension of a 1 wt.% aqueous solution of (1) or (2) against decane; and forms an isotropic surfactant continuous phase (bicontinuous structure). It is also described that the composition has excellent detergency, is safe even when applied to the human body, and is sufficiently eco-friendly. However, the cleansing ability of this composition for the removal of oil stains is unsatisfactory.

In Japanese Patent Application Laid-Open No. 2000-256132, described is a composition containing (1) a silicone oil, (2) a polar oil, (3) a nonionic surfactant, and (4) water, and forming an isotropic surfactant continuous phase (bicontinuous structure). In Japanese Patent Application Laid-Open No. 2000-256124, described is a composition containing (1) a silicone oil, (2) a nonionic surfactant, (3) a hydroxyl-containing water-soluble substance and (4) water, and forming an isotropic surfactant continuous phase (bicontinuous structure). It is described therein that these compositions have good compatibility with the skin and impart smooth touch to the skin when used as a skin care cosmetic; have high makeup removing effects when used as a makeup remover; and have good compatibility with the hair, make the hair luster and provide a good feeling upon use when used as a hair care cosmetic. Their cleansing ability is however not sufficient, and their spreadability or compatibility with stains on the skin is not satisfactory. In addition, it is not easy to remove the detergent compositions and stains with water.

It is disclosed in Japanese Patent Application Laid-Open No. Hei 6-306400 that a composition containing (1) a non polar or a slightly polar solvent, (2) a water soluble or water dispersible low molecular weight amphiphile, and (3) a polar solvent and forming a bicontinuous structure is useful for the removal of fat or tar without any mechanical action. This composition however has not enough cleansing ability for the removal of oil stains. In addition, spreadability or compatibility with stains on the skin is not satisfactory.

Any one of the above-described compositions does not exhibit sufficient cleansing ability for removing both oil stains and water soluble stains. In particular, their cleansing performance is unsatisfactory for the removal of cosmetics such as long-lasting mascara, lipstick and foundation which is durable against sweat or tear and even after meal.

## Disclosure of the Invention

According to the present invention, there is thus provided a skin cleansing composition having (A) an oil component, (B) a hydrophilic nonionic surfactant, (C) a lipophilic amphiphile, (D) a water soluble solvent, and (E) water, and having an isotropic liquid phase exhibiting a bicontinuous structure as definded in claim 1.

## Brief Description of the Drawing

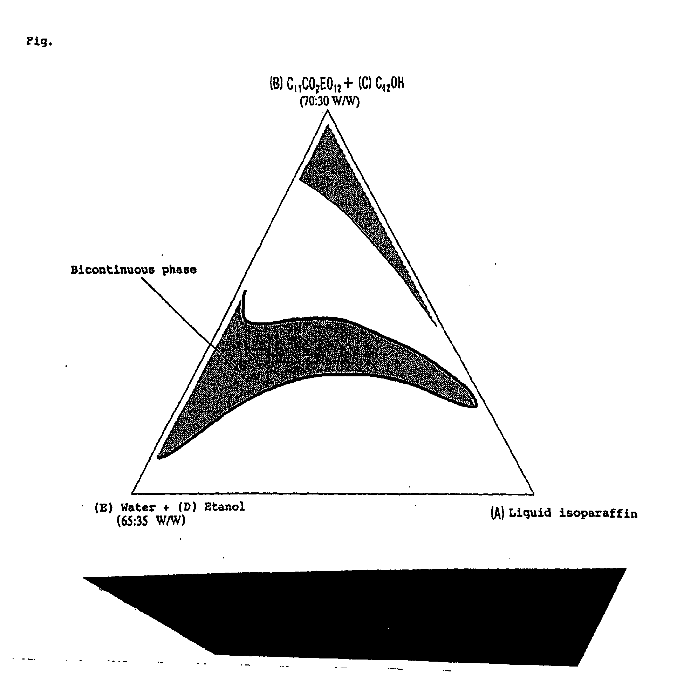

FIG. 1 illustrates one example of a ternary phase diagram of the skin cleansing composition of the present invention consisting of three phases, oil phase/aqueous phase/surfactant phase.

## Detailed Description of the Invention

The present invention relates to a skin cleansing composition having excellent detergency for the removal of oil soluble stains and water soluble stains and having good rinsability.

The present inventors have found that a skin cleansing composition containing the below-described components (A) to (E) and having an isotropic liquid phase with a bicontinuous structure exhibits excellent detergency for the removal of both oil stains and water soluble stains and has good rinsability.

As the oil component used as Component (A) in the present invention, liquid oils ordinarily incorporated in cosmetic compositions can be used. Examples include hydrocarbon oils such as liquid paraffin, liquid isoparaffin and squalane; ester oils such as cholesteryl isostearate, isopropyl palmitate, isopropyl myristate, neopentyl glycol dicaprate, isopropyl isostearate, octadecyl myristate, cetyl 2-ethylhexanoate, isononyl isononanoate, isotridecyl isononanoate, glycerol tri(2-ethylhexaonate), and glycerol tri(caprylate/caprate); ether oils such as an alkyl-1,3-dimethylbutyl ether and nonylphenyl ether; silicone oils, for example, methylcyclopolysiloxanes such as decamethylcyclopentanesiloxane and octamethylcyclotetrasiloxane, methylpolysiloxane and methylphenylpolysiloxane; animal or plant oils such as olive oil; and terpene oil.

The oil component preferably has a viscosity at 25°C of 30 mPa·s or less. The viscosity is measured using a BM type viscometer (manufactured by TOKIMEC INC, measuring conditions: rotor No. 1, 60 rpm).

Of the above-described oil components, liquid paraffin, liquid isoparaffin, neopentyl glycol dicaprate, isopropyl isostearate, cetyl 2-ethylhexanoate, isononyl isononanoate, glycerol tri(caprylate/caprate), alkyl-1,3-dimethylbutyl ethers, decamethylcyclopentasiloxane and octamethylcyclotetrasiloxane are preferred, of which liquid isoparaffin, glycerol tri (caprylate/caprate), alkyl-1, 3-dimethylbutyl ethers and decamethylcyclopentasiloxane are more preferred, with liquid isoparaffin being still more preferred. As the liquid isoparaffin, hydrogenated polyisobutenes are preferred, among which those having a polymerization degree of isobutene ranging from 3 to 6 are more preferred from the viewpoint of cleansing performance in the removal of oil stains.

As Component (A), two or more of the components as exemplified above may be used. Component (A) is incorporated in an amount of from 3 to 80 wt.%, preferably from 5 to 60 wt.% in the whole composition. If the amount is less than 3 wt.%, cleansing ability for the removal of oil stains is not sufficient and rinsability is poor, while the amount exceeding 80 wt.% results in deteriorating the cleansing ability for the removal of water soluble stains.

The hydrophilic nonionic surfactant to be used as Component (B) in the present invention, has an HLB value exceeding 8 and an HLB value not less than 9 is more preferred. The term "HLB value" as used herein indicates the molecular weight of the hydrophilic group in the whole molecular weight of the surfactant. The HLB value of a polyoxyethylene-type nonionic surfactant is determined in accordance with the following Griffin's equation:

$$\text{HLB} = E / 5$$

wherein, E denotes wt.% of the polyoxyethylene contained in the surfactant molecule.

Specific examples of the hydrophilic nonionic surgactants include polyethylene glycol surfactants, for example, polyethylene glycol fatty acid esters such as polyethylene glycol (12) monolaurate, polyethylene glycol alkyl ethers such as polyethylene glycol (20) octyldodecyl ether, polyethylene glycol alkylphenyl ethers such as polyethylene glycol (20) nonylphenyl ether, polyethylene glycol castor oil derivatives such as polyethylene glycol (50) castor oil, polyethylene glycol hydrogenated castor oil derivatives such as polyethylene glycol (60) hydrogenated castor oil monoisolaurate, and polyethylene glycol sorbitan fatty acid esters such as polyethylene glycol (20) sorbitan monostearate; polyglycerin fatty acid esters such as diglycerol monooleate; polyglycerin alkyl ethers such as diglycerin 2-ethylhexyl ether; sucrose fatty acid esters such as sucrose stearate; and alkyl polyglucosides. Of these, the hydrophilic nonionic surfactants whose hydrophobic group has at least 8, especially preferably at least 12 carbon atoms are used because of their good rinsability.

Of the above-described nonionic surfactants, polyethylene glycol fatty acid esters, polyethylene glycol alkyl ethers, polyglycerin fatty acid esters, polyethylene glycol sorbitan fatty acid esters, sucrose fatty acid esters and alkyl polyglucosides are preferred, and polyethylene glycol fatty acid esters, polyethylene glycol alkyl ethers, polyethylene glycol sorbitan fatty acid esters, sucrose fatty acid esters and alkyl polyglucosides are more preferred, because of their high cleansing performance in the removal of oil stains and water soluble stains. Of these, polyethylene glycol fatty acid esters, polyethylene glycol sorbitan fatty acid esters, sucrose fatty acid esters and alkyl polyglucosides are more preferred because of their high safety to the skin. Moreover, polyethylene glycol fatty acid esters and alkyl polyglucosides are still more preferred from the viewpoint of high cleansing performance in removing water soluble stains.

As Component (B), two or more of the nonionic surfactants as exemplified above may be used. Component (B) is added in an amount of from 1 to 45 wt.%, preferably from 1 to 40 wt.% to the total composition. If the amount is less than 1 wt.%, rinsability is deteriorated. On the other hand, if the amount exceeds 45 wt.%, cleansing ability for the removal of oil stains and water soluble stains is deteriorated.

The lipophilic amphiphile used as Component (C) in the present invention, is nonionic surfactants having an HLB value of 8 or less, fatty alcohols having 8 to 25 carbon atoms, fatty acids having 8 to 25 carbon atoms and monoalkylphosphoric acids having an alkyl group of 8 to 25 carbon atoms. These amphiphiles having a hydrophobic group with at least 8 carbon atoms, especially at least 12 carbon atoms are preferred because their cleansing performance in the removal of oil stains and water soluble stains is high.

The nonionic surfactants having an HLB of 8 or less include polyethylene glycol surfactants, for example, ethylene glycol fatty acid esters such as ethylene glycol monostearate, polyethylene glycol fatty acid esters such as polyethylene glycol (2) monostearate, polyethylene glycol alkyl ethers such as polyethylene glycol (5) decyl pentadecyl ether, and polyethylene glycol hydrogenated castor oil derivatives such as polyethylene glycol (5) hydrogenated castor oil monoisolaurate; propylene glycol surfactants, for example, propylene glycol fatty acid esters, polypropylene glycol fatty acid esters, propylene glycol alkyl ethers, polypropylene glycol alkyl ethers and ethylene oxide derivatives of a propylene glycol alkyl ether; glycerin fatty acid esters such as glycerin monoisostearate; glycerin alkyl ethers such as glycerin monoisostearyl ethers; sorbitan fatty acid esters such as sorbitan monostearate; and fatty acid alkanolamides and fatty acid dialkanolamides such as lauric acid diethanolamide. Of these those having an HLB not greater than 6 are preferred because their cleansing performance in removing both oil stains and water soluble stains is high.

Of the above-described nonionic surfactants, polyethylene glycol fatty acid esters, polyethylene glycol alkyl ethers, monoglycerin fatty acid esters, monoglycerin alkyl ethers and sorbitan fatty acid esters are preferred because of their high cleansing performance in removing oil stains and water soluble stains. Of these, monoglycerin fatty acid esters, monoglycerin alkyl ethers and sorbitan fatty acid esters are more preferred because of their high safety to the human skin. Furthermore, monoglycerin alkyl ethers are still more preferred because their cleansing performance in removing both oil stains and water soluble stains is high.

As the fatty alcohols, any monohydric or polyhydric alcohol having a linear or branched, saturated or unsaturated hydrocarbon group having 8 to 25, preferably 12 to 22 carbon atoms can be used. Examples include octanol, lauryl alcohol, myristyl alcohol, isomyristyl alcohol, palmityl alcohol, isopalmityl alcohol, stearyl alcohol, isostearyl alcohol, behenyl alcohol, oleyl alcohol, linoleyl alcohol and linolenyl alcohol. Of these, lauryl alcohol, myristyl alcohol, isomyristyl alcohol, isopalmityl alcohol, isostearyl alcohol and oleyl alcohol are preferred, and lauryl alcohol, myristyl alcohol and isostearyl alcohol are-more preferred from the viewpoint of high cleansing performance in removing both oil stains and water soluble stains.

As the fatty acid, any linear or branched, saturated or unsaturated fatty acid having 8 to 25, preferably 12 to 22 carbon atoms can be used. Examples include lauric acid, myristic acid, isomyristic acid, palmitic acid, isopalmitic acid, stearic acid, behenic acid, isostearic acid, oleic acid, linoleic acid and linolenic acid. Of these, lauric acid, myristic acid, isomyristic acid, isopalmitic acid, isostearic acid, oleic acid, linoleic acid and linolenic acid, especially lauric acid, myristic acid and isostearic acid are preferred because their cleansing performance in removing both oil stains and water soluble stains is high.

As the monoalkylphosphoric acid, those having a linear or branched alkyl group of 8 to 25, preferably 12 to 22 carbon atoms are preferred. Examples include monolaurylphosphoric acid, monomyristylphosphoric acid, monopalmitylphosphoric acid, monostearylphosphoric acid, monobehenylphosphoric acid, monoisostearylphosphoric acid and mono-2-hexyldecylphosphoric acid. Of these, monolaurylphosphoric acid, monomyristylphosphoric acid and mono-2-hexyldecylphosphoric acid are preferred and monolaurylphsophoric acid and monomyristylphosphoric acid are more preferred because their cleansing performance for the removal of both oil stains and water soluble stains is high.

As Component (C), two or more of the amphiphiles as exemplified above can be used. Component (C) is added in an amount of from 1 to 45 wt.%, preferably 1 to 40 wt.%, based on the whole composition. If the amount is less than 1 wt.%, cleansing ability and rinsability are deteriorated. On the other hand, if the amount exceeds 45 wt.%, cleansing ability for the removal of water soluble stains and rinsability are deteriorated.

In the present invention, the hydrophilic nonionic surfactant (B) and the lipophilic amphiphile (C) are incorporated preferably at a (B)/(C) weight ratio of from 0.5 to 8, because good rinsability and high cleansing performance for removing both oil stains and water soluble stains can be attained at this ratio.

As the water soluble solvent (D) used in the present invention, preferred are monohydric or polyhydric alcohols having 1 to 6 carbon atoms, polyethylene glycols, polypropylene glycols, saccharides and water soluble fatty acids.

As Component (D), those capable of improving the hydrophilic property of the hydrophilic nonionic surfactant (B) and that of the lipophilic amphiphile (C) are preferred. The property capable of improving the hydrophilic property of the hydrophilic nonionic surfactant (B) and that of the lipophilic amphiphile (C) means, for example, a property of raising the cloud point of the nonionic surfactant by adding the Component (D), which is described in Sagitani, et al., "Oleo Science", Vol. 33, No. 3, p. 156-161 (1984).

The monohydric alcohols having 1 to 6 carbon atoms include ethanol, propanol, isopropanol, butanol and isobutanol, while the polyhydric alcohols include ethylene glycol, propylene glycol, isoprene glycol, 1,3-butylene glycol, hexylene glycol, trimethylolpropane, glycerin and sorbitol. Of these, ethanol, propanol and isopropanol are preferred, and ethanol is more preferred as the monohydric alcohol; and propylene glycol, isoprene glycol, 1,3-butylene glycol and hexylene glycol are preferred, and isoprene glycol and hexylene glycol are more preferred as the polyhydric alcohol, because their cleansing performance in removing both oil stains and water stains is high.

As the polyethylene glycols or polypropylene glycols, polyethylene glycols having a molecular weight not greater than 1000 and polypropylene glycols having a molecular weight not greater than 200 can be used. Examples include diethylene glycol, dipropylene glycol, diethylene glycol monoethyl ether and diethylene glycol monobutyl ether. Of these dipropylene glycol, diethylene glycol monoethyl ether, and diethylene glycol monobutyl ether are preferred, and dipropylene glycol and diethylene glycol monoethyl ether are more preferred, because their cleansing performance in the removal of both oil stains and water soluble stains is high.

The saccharides include, for example, erythritol, pentaerythritol, methyl glucoside, ethyl glucoside, polyoxyethylene methyl glucoside and polyoxypropylene methyl glucoside. Among them, alkyl glucosides having an alkyl chain of 2 or less carbon atoms are preferred. Of them, methyl glucoside, ethyl glucoside, polyoxyethylene methyl glucoside and polyoxypropylene methyl glucoside are preferred, and polyoxyethylene methyl glucoside and polyoxypropylene methyl glucoside are more preferred because high cleansing performance in the removal of both oil stains and water soluble stains can be attained when they are used.

The water soluble fatty acids include fatty acids having 1 to 6 carbon atoms such as acetic acid, propionic acid, and butanoic acid. Acetic acid and propionic acid are preferred, and propionic acid is more preferred because high cleansing performance in removing both oil stains and water soluble stains can be attained when they are used.

As Component (D), two or more of the solvents as exemplified above can be used and Component (D) is added in an amount of 3 to 80 wt.%, preferably 5 to 70 wt.% based on the whole composition. If the amount is less than 3 wt.%, cleansing ability for the removal of oil stains is deteriorated. On the other hand, if the amount exceeds 80 wt.%, cleansing performance for the removal of oil stains is deteriorated.

For attaining high cleansing performance in removing both oil stains and water soluble stains, a weight ratio (D) / ((B) + (C)), that is, a ratio of Component (D) to the sum of the hydrophilic nonionic surfactant (B) and the lipophilic amphiphile (C) is preferably 1 or greater.

Water as Component (E) is added in an amount of from 3 to 80 wt.%, preferably from 5 to 75 wt.% based on the total composition. If the amount is less than 3 wt.%, cleansing ability for the removal of water soluble stains and rinsability are deteriorated, while that exceeding 80 wt.% results in deteriorating cleansing ability for the removal of oil stains.

In the skin cleansing composition of the present invention, it is possible to incorporate components ordinarily employed for cleansing compositions, for example, thickeners, bactericides, humectants, colorants, antiseptics, feel improvers, perfumes, anti-inflammatory agents, whitening agents, antiperspirants and ultraviolet absorbers as needed.

The skin cleansing composition of the present invention can be prepared by mixing all the components, irrespective of the mixing order. A raw material which is in the solid form at ambient temperature is dissolved in other components, if necessary, by heating.

The skin cleansing composition of the present invention can be used as a cleansing agent for body or face. Furthermore, it can be impregnated into a substrate like a non-woven fabric to provide a skin cleansing sheet article for wiping and removing makeup stains or sebum.

The skin cleansing composition of the present invention has an isotropic liquid phase exhibiting a bicontinuous structure. The term "isotropic liquid phase exhibiting a bicontinuous structure" means a transparent or translucent low-viscosity solution which has an aqueous phase and an oil phase each existing continuously and is optically isotropic. Specifically, it means a middle phase (or D phase) and a sponge phase (or $L_3$ phase).

The isotropic liquid phase exhibiting a bicontinuous structure of the skin cleansing composition according to the present invention can be confirmed by the observation of appearance, observation by an optical polarizing microscope, drawing of a phase diagram, measurement of a self diffusion coefficient by NMR, measurement of electrical conductivity, fluorescent probe method using a fluorescent dye, or observation by an electronic microscope (such as TEM or SEM) in accordance with freeze fracture replica method.

It is possible to distinguish a solution having an isotropic liquid phase exhibiting a bicontinuous structure from the other solution by the observation of appearance, because the former one is in the low-viscosity solution form with a transparent appearance. It is also possible to confirm the isotropic form by arranging two polarizing plates with their polarizing directions perpendicular to each other, and placing, between them, a sample charged in a transparent container. When no light transmission is observed, the solution is confirmed to be isotropic. Through observation by an optical polarizing microscope, the solution can be confirmed to be isotropic if no light transmission is observed at an angle of a polarizing plate set at 90 degree.

Upon confirmation by using a pseudo phase diagram for an aqueous phase (water and water solvent), an oil phase (oil component) and a surfactant phase (hydrophilic nonionic surfactant and lipophilic amphiphile), the solution is confirmed to be isotropic if it is in the isotropic liquid form on the phase diagram and is not in a region extending continuously from the apex of the aqueous phase or oil phase. This method is not always applied depending on the substances employed, composition of the aqueous phase or composition of the surfactant phase.

The measurement of a self diffusion coefficient by NMR is a method as described in detail in B. Lindman, et al., "J. Colloid Interface Sci. 83, 569 (1981)".

The measurement of electrical conductivity is a method as described in detail in M. Clausse, et al., "Microemulsion Systems", Marcel Dekker, New York, 387(1987).

The measurement in accordance with the fluorescent probe method using a fluorescent dye is a method as described in detail in B.K. Mishra, et al., "Colloid Surface", 56, 229 (1991).

According to a freeze fracture replica method using an electronic microscopy, an image of a continuous phase formed by an aqueous phase and an oil phase can be observed. Specifically, an entirely rounded portion, and a structure in which flattish portions are entangled in the network form, or a layered structure in which flattish portions exist at random are observed. By this observation, the solution can be confirmed to be not a microemulsion in which only an aqueous phase or an oil phase forms a continuous phase.

FIG. 1 is a ternary phase diagram of oil phase (Component (A))/aqueous phase (Components (D) and (E))/ surfactant phase (Components (B) and (C)) when the skin cleansing composition of the present invention comprises (A) liquid isoparaffin, (B) polyethylene glycol monolaurate ("EMANON® 1112", product of Kao Corp-), (C) lauryl alcohol, (D) ethanol and (E) water. The region of an isotropic liquid phase exhibiting a bicontinuous structure formed by these components is illustrated as a colored portion in FIG. 1. In the figure, $C_{11}CO_2EO_{12}$ denotes polyethylene glycol (12 moles) monolaurate and $C_{12}OH$ means lauryl alcohol.

**Examples**

Example 1-16

Skin cleansing compositions having the compositions as shown in Table 1 were prepared and their cleansing performance in removing oil mascara and aqueous mascara, and rinsing performance were evaluated. The results are also shown in Table 1. With regards to the oil components used in Examples, a viscosity at 25°C is appended.

(Preparation Process)

Skin cleansing compositions were prepared by uniformly mixing all the raw materials. Liquid materials at normal temperature were used as they were and those materials solid at normal temperature were used after dissolution.

Observation of the appearance has revealed that the skin cleansing compositions thus obtained each had an isotropic liquid phase. It can be confirmed by the measurement of a self diffusion coefficient by NMR that they have a bicontinuous structure.

(Evaluation method)

(1) Cleansing performance:

After 10 mg of a waterproof mascara ("Full and Soft Mascara, waterproof, 01 Black", trade name; product of MAYBELLINE) was applied to the arm, about 100 mg of the skin cleansing composition was weighed and applied thereon. The arm was massaged therewith 10 times at a constant force and speed, followed by rinsing with water at 30°C. The cleansing performance in the removal of the oil mascara was then evaluated based on the below-described criteria. The cleansing performance in the removal of an aqueous mascara was evaluated similarly by using an aqueous type mascara ("PIEDS NUS® More-glamorous Mascara BK999"; product of Shiseido).

A: Almost all the mascara was cleansed away.
B: A large portion of the mascara was cleansed away.
C: A small portion of the mascara was cleansed away.
D: The mascara was hardly cleansed away.

(2) Rinsing performance

The rinsability was evaluated after about 2 g of the skin cleansing composition was applied to the forearm and then it was rinsed off with warm water.

A: Sliminess disappears and the composition is easily rinsed off.
B : Sliminess remains and the composition is not easily rinsed off.
C: Greasiness continues to remain on the skin and cannot be rinsed off.

Table 1

| | | Example products | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| A | Hydrogenated polyisobutene (polymerization degree: 5); 15mPa·s | 10.0 | | | | | | | 10.0 | 10.0 | 10.0 | 10.0 | 10.0 | 55.0 | 10.0 | 5.0 | |
| | Hydrogenated polyisobutene (polymerization degree: 3); 3mPa·s | | 15.0 | | | | 15.0 | 15.0 | | | | | | | | | 30.0 |
| | Glycerol tri(caprylate/caprate); 27 mPa·s | | | 5.0 | | 7.0 | | | | | | | | | | | |
| | Decamethylcyclopentanesiloxane; 3 mPa·s ("Silicone SH245®", product of Toray Dow Coming Silicone Co., Ltd.) | | | | 5.0 | | | | | | | | | | | | |
| B | Polyethylene glycol monolaurate (HLB=13.7; "EMANON® 1112; product of Kao Corp.) | 14.0 | 3.7 | 20.0 | 20.0 | | | | | 14.0 | 8.0 | 10.0 | 14.0 | 8.0 | 14.0 | | 7.4 |
| | Polyoxyethylene decyl totradecyl ether (HLB=11; "EMALEX® 2415", product of Nihon Emulsion Co., Ltd.) | | | | | 20.0 | | | | | | | | | | | |
| | Alkyl glucoside (HLB=17; "MYDOL® 10", product of Kao Corp.) | | | | | | 8.0 | | | | | | | | | | |
| | Sucrose laurate (HLB=16; "SURFHOPE® SE COSME C1216, product of Mitsubishi-Kagaku Foods Corp) | | | | | | | 3.1 | | | | | | | | | |
| | Polyoxyethylene sorbitan monostearate (HLB=14.9; -RHEODOL SUPER® TW-S120"; product of Kao Corp.) | | | | | | | | | 10.0 | | | | | | 7.5 | |

EP 1 433 476 B2

(continued)

| | | Example products | | | | | | | | | | | | | | | |
|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 |
| C | Lauryl alcohol | 6.0 | | 3.0 | 10.0 | 3.0 | | | 10.0 | | | 6.0 | 6.0 | | 6.0 | 7.5 | |
| | Lauric acid | | | | | | | | | 6.0 | | | | | | | |
| | Monolaurylphosphoric acid | | | | | | | | | | 12.0 | | | | | | |
| | Isostearyl glyceryl ether (HLB=5.3; "PENETOL® GE-IS"; product of Kao Corp.) | | 1.3 | | | | 2.0 | 1.9 | | | | 10.0 | | 12.0 | | 40.0 | 2.6 |
| D | Ethanol | 25.0 | 5.0 | 40.0 | 35.0 | 38.0 | | 15.0 | 35.0 | 25.0 | 20.0 | 35.0 | | | | 40.0 | 5.0 |
| | Dipropylene glycol | | | | | | | | | | | | 40.0 | | | | |
| | Isoprene glycol | | 5.0 | | | | 15.0 | | | | | | | 20.0 | 50.0 | | 5.0 |
| E | Water | 45.0 | 70.0 | 30.0 | 30.0 | 32.0 | 60.0 | 60.0 | 35.0 | 45.0 | 50.0 | 35.0 | 30.0 | 5.0 | 20.0 | 40.0 | 65.0 |
| Cleansing performance in removing oily mascara | | A | A | B | B | B | B | B | A | A | A | A | A | A | A | A | B |
| Cleansing performance in removing aqueous mascara | | A | A | A | A | B | A | B | B | A | A | A | A | A | A | B | A |
| Rinsability | | A | A | A | A | A | A | A | A | A | A | A | A | A | A | A | A |

Example 17, Comparative Example 1-16

In a similar manner to Example 1, skin cleansing compositions having the compositions as shown in Tables 2 and 3 were prepared and their cleansing performance in removing oil mascara and aqueous mascara, and rinsability were evaluated. The phase state of each of the resulting skin cleansing compositions was confirmed by observing the appearance and measuring a self diffusion coefficient by NMR. The results are shown in Tables 2 and 3.

Table 2

| Component (wt.%) | | Example product | Comparative product | | | | | |
|---|---|---|---|---|---|---|---|---|
| | | 1 | 1 | 2 | 3 | 4 | 5 | 6 |
| A | Hydrogenated polyisobutene (polymerization degree: 5); 15 mPa·s | 10.0 | 10.0 | 10.0 | - | 10.0 | 10.0 | 10.0 |
| | Decamethylcyclopentanesiloxane; 3 mPa·s | | | | 10.0 | | | |
| B | Polyethylene glycol monolaurate (HLB=13.7; "EMANON® 1112", product of Kao Corp.) | 14.0 | | 14.0 | 14.0 | 14.0 | 14.0 | 18.0 |
| C | Lauryl alcohol | 6.0 | 6.0 | | | 6.0 | 6.0 | 2.0 |
| D | Ethanol | 25.0 | 25.0 | 25.0 | 25.0 | | 70.0 | 3.5 |
| E | Water | 45.0 | 59.0 | 51.0 | 51.0 | 70.0 | | 66.5 |
| Phase state | | Isotropic liquid phase exhibiting a biconlinuous structure | Phase separation | Phase separation | Phase separation | Phase separation | Single phase | O/W micro-emulsion |
| Cleansing performance in removing oil mascara | | A | D | D | D | D | C | D |
| Cleansing performance in removinq aqueous mascara | | A | C | B | B | B | B | B |
| Rinsing performance | | A | C | C | C | C | A | A |

Table 3

| Component (wt.%) | | Comparative product | | | | | | | | | |
|---|---|---|---|---|---|---|---|---|---|---|---|
| | | 7 | 8 | 9 | 10 | 11 | 12 | 13 | 14 | 15 | 16 |
| A | Hydrogenated polyisobutene (polymerization degree: 5); 15 mPa·s | 85.0 | 1.0 | 10.0 | 10.0 | 10.0 | 10.0 | 7.0 | 20.0 | 6.0 | 30.0 |
| B | Polyethylene glycol monolaurate (HLB=13.7; "EMANON® 1112". product of Kao Corp.) | 3.0 | 10.0 | 50.0 | 0.5 | 14.0 | 10.0 | 2.0 | 14.0 | 2.0 | 14.0 |
| C | Lauryl ether | 3.0 | 10.0 | 6.0 | 10.0 | 50.0 | 0.5 | 2.0 | 6.0 | 2.0 | 6.0 |
| D | Ethanol | 4.0 | 25.0 | 10.0 | 25.0 | 6.0 | 35.0 | 85.0 | 1.0 | 5.0 | 49.0 |
| E | Water | 5.0 | 54.0 | 24.0 | 54.5 | 20.0 | 44.5 | 4.0 | 59.0 | 85.0 | 1.0 |
| Phase state | | Separation into 2 phases | Separation into 2 phases | Separation into 2 phases | Separation into 2 phases | Separation into 2 phases | Separation into 2 phases | Separation into 2 phases | Separation into 2 phases | Separation into 2 phases | Separation into 2 phases |
| Cleansing performance in removing oil mascara | | B | D | C | C | C | D | C | D | D | C |
| Cleansing performance in removing aqueous mascara | | D | B | B | B | D | D | B | B | B | D |
| Rinsing performance | | C | C | C | C | C | C | C | A | C | C |

**Industrial Applicability**

The skin cleansing composition of the present invention exhibits excellent detergency for the removal of both oil stains and water soluble stains and at the same time, has good rinsability.

**Claims**

1. A skin cleansing composition comprising

(A) 3 to 80 wt.% of an oil component,
(B) 1 to 45 wt.% of a hydrophilic nonionic surfactant,
(C) 1 to 45 wt.% of a lipophilic amphiphile,
(D) 3 to 80 wt.% of a water soluble solvent and
(E) 3 to 80 wt.% of water,
and having an isotropic liquid phase exhibiting a bicontinuous structure,
wherein the hydrophilic nonionic surfactant (B) has an HLB value of more than 8 and has a hydrophobic group with 8 or more carbon atoms, and
wherein the lipophilic amphiphile (C) is selected from nonionic surfactants having an HLB value of 8 or less, fatty alcohols having 8 to 25 carbon atoms, fatty acids having 8 to 25 carbon atoms and monoalkylphosphoric acids having 8 to 25 carbon atoms.

2. The skin cleansing composition of Claim 1, wherein the oil component (A) has a viscosity at 25 °C of 30 mPa.s or less.

3. The skin cleansing composition of Claim 1 or 2, wherein the oil component (A) is selected from liquid paraffin, liquid isoparaffin, neopentyl glycol dicaprate, isopropyl isostearate, cetyl 2-ethylhexanoate, isononyl isononanoate, glycerol tri(caprylate/caprate), alkyl-1,3-dimethylbutyl ethers, decamethylcyclopentasiloxane and octamethylcyclotetrasiloxane.

4. The skin cleansing composition of any one of Claims 1 to 3, wherein the oil component (A) is hydrogenated polyisobutene which has a polymerization degree of from 3 to 6.

5. The skin cleansing composition of any one of Claims 1 to 4, wherein the hydrophilic nonionic surfactant (B) is selected from polyethylene glycol fatty acid esters, polyethylene glycol alkyl ethers, polyethylene glycol sorbitan fatty acid esters, sucrose fatty acid esters and alkyl polyglucosides.

6. The skin cleansing composition of any one of Claims 1 to 5, wherein the hydrophilic nonionic surfactant (B) is selected from polyethylene glycol fatty acid esters and alkyl polyglucosides.

7. The skin cleansing composition of any one of Claims 1 to 6, wherein the water soluble solvent (D) is selected from ethanol, isoprene glycol, hexylene glycol, dipropylene glycol, diethylene glycol monoethyl ether, polyoxyethylene methyl glucoside, polyoxypropylene methyl glucoside and propionic acid.

8. The skin cleansing composition of any one of Claims 1 to 7, wherein, the weight ratio of the water soluble solvent (D) to the sum of the hydrophilic nonionic surfactant (B) and lipophilic amphiphile (C), (D)/((B)+(C)), is 1 or greater.

**Patentansprüche**

1. Hautreinigungszusammensetzung, umfassend

(A) 3 bis 80 Gew.-% einer Ölkomponente,
(B) 1 bis 45 Gew.-% eines hydrophilen nicht-ionischen Tensides,
(C) 1 bis 45 Gew.-% eines lipophilen Amphiphils,
(D) 3 bis 80 Gew.-% eines wasserlöslichen Lösungsmittels und
(E) 3 bis 80 Gew.-% Wasser, und
mit einer isotropen Flüssigphase, die eine bikontinuierliche Struktur entfaltet,
worin das hydrophile nicht-ionische Tensid (B) einen HLB-Wert von mehr als 8 aufweist und eine hydrophobe Gruppe mit 8 oder mehr Kohlenstoffatomen hat, und
worin das lipophile Amphiphil (C) ausgewählt ist aus nicht-ionischen Tensiden mit einem HLB-Wert von 8 oder weniger, Fettalkoholen mit 8 bis 25 Kohlenstoffatomen, Fettsäuren mit 8 bis 25 Kohlenstoffatomen und Monoalkylphosphorsäuren mit 8 bis 25 Kohlenstoffatomen.

2. Hautreinigungszusammensetzung nach Anspruch 1, worin die Ölkomponente (A) eine Viskosität von 30 mPa·s oder weniger bei 25°C hat.

3. Hautreinigungszusammensetzung nach Anspruch 1 oder 2, worin die Ölkomponente (A) ausgewählt ist aus flüssigem Paraffin, flüssigem Isoparaffin, Neopentylglycoldicaprat, Isopropylisostearat, Cetyl-2-ethylhexanoat, Isononylisononanoat, Glyceroltri(caprylat/caprat), Alkyl-1,3-dimethylbutylethern, Decamethylcyclopentasiloxan und Octamethylcyclotetrasiloxan.

4. Hautreinigungszusammensetzung nach einem der Ansprüche 1 bis 3, worin die Ölkomponente (A) hydriertes Polyisobuten mit einem Polymerisationsgrad von 3 bis 6 ist.

5. Hautreinigungszusammensetzung nach einem der Ansprüche 1 bis 4, worin das hydrophile nicht-ionische Tensid (B) ausgewählt ist aus Polyethylenglycolfettsäureestern, Polyethylenglycolalkylestern, Polyethylenglycolsorbitanfettsäureestern, Succrosefettsäureestern und Alkylpolyglucosiden.

6. Hautreinigungszusammensetzung nach einem der Ansprüche 1 bis 5, worin das hydrophile nicht-ionische Tensid (B) ausgewählt ist aus Polyethylenglycolfettsäureestern und Alkylpolyglucosiden.

7. Hautreinigungszusammensetzung nach einem der Ansprüche 1 bis 6, worin das wasserlösliche Lösungsmittel (D) ausgewählt ist aus Ethanol, Isoprenglycol, Hexylenglycol, Dipropylenglycol, Diethylenglycolmonoethylether, Polyoxyethylenmethylglucosid, Polyoxypropylenmethylglucosid und Propionsäure.

8. Hautreinigungszusammensetzung nach einem der Ansprüche 1 bis 7, worin das Gewichtsverhältnis des wasserlöslichen Lösungsmittels (D) zu der Summe des hydrophilen nicht-ionischen Tensides (B) und des lipophilen Amphiphils (C), (D)/((B)+(C)), 1 oder mehr ist.


**Revendications**

1. Composition de nettoyage pour la peau comprenant

(A) 3 à 80% en poids d'un composant huileux,
(B) 1 à 45% en poids d'un tensioactif non ionique hydrophile,
(C) 1 à 45% en poids d'un composé amphiphile lipophile,
(D) 3 à 80% en poids d'un solvant soluble dans l'eau et
(E) 3 à 80% en poids d'eau,
et ayant une phase liquide isotrope présentant une structure bicontinue,
dans laquelle le tensioactif non ionique hydrophile (B) a une valeur HLB supérieure à 8 et contient un groupe hydrophobe comportant 8 atomes de carbone ou plus, et
dans laquelle le composé amphiphile lipophile (C) est choisi parmi les tensioactifs non ioniques ayant une valeur HLB de 8 ou moins, les alcools gras comportant 8 à 25 atomes de carbone, les acides gras comportant 8 à 25 atomes de carbone et les acides monoalkylphosphoriques comportant 8 à 25 atomes de carbone.

2. Composition de nettoyage pour la peau selon la revendication 1, dans laquelle le composant huileux (A) a une viscosité à 25°C de 30 mPa.s ou moins.

3. Composition de nettoyage pour la peau selon la revendication 1 ou 2, dans laquelle le composant huileux (A) est choisi parmi la paraffine liquide, l'isoparaffine liquide, le dicaprate de néopentylglycol, l'isostéarate d'isopropyle, le 2-éthylhexanoate de cétyle, l'isononanoate d'isononyle, le tri(caprylate/caprate) de glycérol, les alkyl-1,3-diméthylbutyléthers, le décaméthylcyclopentasiloxane et l'octaméthylcyclotétrasiloxane.

4. Composition de nettoyage pour la peau selon l'une quelconque des revendications 1 à 3, dans laquelle le composant huileux (A) est du polyisobutène hydrogéné qui a un degré de polymérisation de 3 à 6.

5. Composition de nettoyage pour la peau selon l'une quelconque des revendications 1 à 4, dans laquelle le tensioactif non ionique hydrophile (B) est choisi parmi les esters d'acides gras de polyéthylèneglycol, les éthers alkyliques de polyéthylèneglycol, les esters d'acides gras de sorbitan de polyéthylèneglycol, les esters d'acides gras de saccharose et les alkylpolyglucosides.

6. Composition de nettoyage pour la peau selon l'une quelconque des revendications 1 à 5, dans laquelle le tensioactif non ionique hydrophile (B) est choisi parmi les esters d'acides gras de polyéthylèneglycol et les alkylpolyglucosides.

7. Composition de nettoyage pour la peau selon l'une quelconque des revendications 1 à 6, dans laquelle le solvant soluble dans l'eau (D) est choisi parmi l'éthanol, l'isoprèneglycol, l'hexylèneglycol, le dipropylèneglycol, l'éther monoéthylique de diéthylèneglycol, le polyoxyéthylèneméthylglucoside, le polyoxypropylèneméthylglucoside et l'acide propionique.

8. Composition de nettoyage pour la peau selon l'une quelconque des revendications 1 à 7, dans laquelle le rapport en poids du solvant soluble dans l'eau (D) à la somme du tensioactif non ionique hydrophile (B) et du composé amphiphile lipophile (C), (D)/((B) + (C)) est de 1 ou plus.

EP 1 433 476 B2

Fig.

(B) $C_{11}CO_2EO_{12}$ + (C) $C_{12}OH$
(70:30 W/W)

Bicontinuous phase

(E) Water + (D) Etanol
(65:35 W/W)

(A) Liquid isoparaffin

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP HEI6293617 B
- JP 2002020791 A
- JP 2000256132 A
- JP 2000256124 A
- JP HEI6306400 B

### Non-patent literature cited in the description

- *Journal of Oleo Science,* 1994, vol. 43 (2), 131-136
- **SAGITANI et al.** *Oleo Science,* 1984, vol. 33 (3), 156-161
- **B. LINDMAN et al.** *J. Colloid Interface Sci.,* 1981, vol. 83, 569
- **M. CLAUSSE et al.** Microemulsion Systems. Marcel Dekker, 1987, 387
- **B.K. MISHRA et al.** *Colloid Surface,* 1991, vol. 56, 229